# EUROPEAN PATENT APPLICATION

(11) **EP 1 365 018 A1**
(43) Date of publication of application: **26.11.2003**
(21) Application number: 02291273.7
(22) Date of filing: 23.05.2002
(51) Int. Cl.: C12N 5/06, A61K 39/395, A61K 38/17, A61K 31/7088, G01N 33/53, A61K 35/30, A61P 25/28, A61P 25/02

(54) **CD100 semaphorin in myelination**

(71) Applicant: INSTITUT NATIONAL DE LA SANTE ET DE LA RECHERCHE MEDICALE (INSERM) (E.P.S.T.), 75013 Paris (FR)
(72) Inventor: Chedotal, Alain, 92340 Bourg la Reine (FR); Moreau-Fauvarque, Caroline, 92190 Meudon (FR)
(74) Representative: Bernasconi, Jean

(57) **Abstract**

The invention relates to the use of a modulator of the CD100 semaphorin or of a modulator of a CD100 semaphorin receptor for the preparation of a medicament useful in the prevention of demyelination and/or for enhancement of axonal myelination.

## Description

The present invention relates to the role of CD100 semaphorin in myelination and demyelination processes, and to resulting therapeutic and diagnostic methods.

The semaphorins are membrane-bound and secreted proteins which have been identified in multiple animal species (Raper, 2000). To date, about 20 distinct semaphorin proteins have been found in vertebrates sharing a highly conserved domain of ∼500 amino acids, the Sema domain. They can be grouped into 7 distinct classes based on sequence homologies, and are all expressed in the embryonic and adult nervous system. All secreted Class 3 semaphorins bind receptors that consist of a member of the neuropilin family as ligand-binding subunit and a member of the plexin family as signal transducing subunit (Fujisawa and Kitsukawa, 1998 ; Tamagnone and Comoglio, 2000). Several membrane-bound semaphorins have been also shown to use the plexins as their receptors. There are at least nine plexins in humans, grouped into four subfamilies (A-D) according to sequence similarity. Several semaphorin peptides are also described in U.S. patent No. 6,013,781, whereas plexins are disclosed in the international patent application WO 01/01420.

CD100 was originally identified in the immune system using monoclonal antibodies (Hérold et al., 1995). The identification of CD100 is described in the international patent application WO 97/17368 as well. CD100 is a 150 kDa homodimeric transmembrane protein expressed on the majority of haematopoietic cells, including B and T lymphocytes and monocytes (Delaire et al., 1998). CD100 expression is the strongest on activated T lymphocytes. The molecular cloning of CD100 indicated that it belongs to the semaphorin family (Hall et al., 1996; Furuyama et al., 1997). CD100 (also called Sema4D, or M-Sema G) is a class 4 semaphorin, that comprises transmembrane molecules containing a C2 class immunoglobulin-like (Ig) domain following the Sema domain. The juxtamembrane domain of CD100 is unique and does not show any homology with other known proteins.

CD100 stimulation by monoclonal antibodies has shown that CD100 is involved in T-cell activation (Hérold et al., 1995). CD100 also induces B lymphocytes to aggregate and improves their viability *in vitro*. In addition, CD100 enhances antibody production *in vivo,* as well as B cell responses *in vitro* (Kumanogoh et al., 2000). CD100 can therefore function as a ligand in the immune system.

So far, two distinct receptors for CD100 have been characterized. The first one, Plexin-B1 is a high affinity receptor for CD100 (Tamagnone et al., 1999). Plexin-B1 is widely expressed in fetal and adult tissue (Maestrini et al., 1996), suggesting a role for CD100-plexin-B1 interaction in the development of plexin-B1 expressing tissues. The second one is CD72, a functional lower affinity receptor for CD100, expressed on lymphocytes (which do not express plexin-B1). Therefore, CD100 may differentially use plexin-B 1 or CD72 as a receptor, depending on the tissue.

Despite an increasing number of studies on the role of semaphorins in axon guidance and neuronal migration (Raper, 2000), their function in the adult nervous system is still poorly understood. The expression pattern of almost all semaphorins in the developing and adult brain has been reported and so far they have been only detected in neurons.

The analysis of CD100 mRNA expression by northern blots and *in situ* hybridization indicated that CD100 is broadly expressed in haematopoietic and nonhaematopoietic tissues, such as skeletal muscles, testes and brain (Hall et al., 1996; Furuyama et al., 1997). Furuyama et al. (1997), have also shown by *in situ* hybridization that mouse CD100/Sema4D mRNA is expressed in the embryonic CNS. However, CD100 expression in the CNS at later stages of development and in the adult brain has never been reported.

The present inventors have performed an extensive study of the expression pattern of many secreted and transmembrane semaphorins. They have shown that the class 4 semaphorin Sema4D/CD100 and one of its receptor Plexin-B3 are expressed in oligodendocytes during myelination.

On that basis they now propose to modulate CD100 or a receptor thereof to prevent demyelination and/or to enhance axonal myelination.

As myelin has been shown to contain proteins such as NogoA and MAG, that inhibit axonal regeneration following injury, and since CD100 semaphorin is involved in myelination and demyelination processes, the invention also makes it possible to stimulate axonal regeneration by modulating CD100 semaphorin or a receptor thereof.

### General definitions

In the context of the present invention, the terms "CD100 semaphorin", "CD100", or "Sema4D" refer to a native semaphorin 150 kDa homodimeric transmembrane protein, as above described, including the human form (GENBANK Access number: U60800), or any splice variant, or variant of other species, including rodents (GENBANK Acess number for mouse U69535), dogs, cats, monkeys, etc.

"A CD100 semaphorin receptor" includes CD72, and plexins, such as plexin B1 and plexin B3.

"Modulators of the CD100 semaphorin" or "CD100 modulators" include activators or inhibitors of the CD100 semaphorin. Similarly "modulators of a CD100 receptor" include activators or inhibitors of the CD100 semaphorin receptor.

"Activators of the CD100 semaphorin" ("CD100 activators"), or "activators of a CD100 receptor" ("CD100 receptor activators") are meant to include any substance that triggers or enhances the expression or biological activity of CD100 or of a CD100 receptor, respectively. Preferred activators directly interact with CD100 semaphorin, a CD100 receptor or with a binding partner. "Binding partners" are proteins or polypeptides that directly interact with CD100, such as serine kinases, protein tyrosine phosphatases (*e.g.* CD45). Activators also encompass any substance that augments the amount of CD100 semaphorin or one of its receptors, *e.g.* by enhancement of the transcription. Exogenous CD100 or CD100 receptor nucleic acid or protein may also serve to augment the amount of CD100 semaphorin or one of its receptors.

"Inhibitors of the CD100 semaphorin" ("CD100 inhibitors"), or "inhibitors of a CD100 receptor" ("CD100 receptor inhibitors") are meant to include any substance that reduces or blocks the expression or biological activity of CD100 or of a CD100 receptor, respectively. Preferred inhibitors directly interact with CD100 semaphorin, or a CD100 receptor or with a binding partner. Such inhibitors include any substance that prevents CD100 from binding to at least one of its receptor.

The "biological activity" of the CD100 semaphorin or of its receptors refers for example to the activation of plexin-B receptors inducing an inhibition of Rac-induced p21-activated kinase (PAK) (Vikis HG, et al, 2002) and more generally to the action of CD100 in modulation of cell migration, of axon outgrowth, of cell differentiation, and formation of myelin.

In accordance with the present invention there may be employed conventional molecular biology, microbiology, and recombinant DNA techniques within the skill of the art. Such techniques are explained fully in the literature. See, e.g., Sambrook et al., (1989) ; B.D. Hames & S.J. Higgins, (1984) ; R.I. Freshney, (1986) ; Perbal, (1984) ; F.M. Ausubel et al. (1994).

The nucleic acid used in the context of the present invention can be a deoxyribonucleic acid (DNA) or a ribonucleic acid (RNA). Among DNAs, possible alternatives include a complementary DNA (cDNA), a genomic DNA (gDNA), a hybrid sequence or a synthetic or semi-synthetic sequence. A further possibility is a nucleic acid modified chemically, for example, for the purpose of increasing its resistance to nucleases, its cell penetration or cell targeting, its therapeutic efficacy, and the like. These nucleic acids can be of human, animal, plant, bacterial, viral, synthetic and the like, origin. They may be obtained by any technique known to a person skilled in the art, and in particular by screening of libraries, by chemical synthesis or alternatively by mixed methods including the chemical or enzymatic modification of sequences obtained by screening of libraries. As mentioned later, they can, moreover, be incorporated in vectors such as plasmid, viral or chemical vectors.

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene", also called a "structural gene" means a DNA sequence that codes for or corresponds to a particular sequence of amino acids, which comprises all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription.

A coding sequence is "under the control of" or "operatively associated with" expression (i.e. transcription and/or translation) control sequences in a cell when RNA polymerase transcribes the coding sequence into RNA, particularly mRNA, which is then trans-RNA spliced (if it contains introns) and translated into the protein encoded by the coding sequence.

A "promoter" or "promoter sequence" is a DNA regulatory region capable of binding RNA polymerase in a cell and initiating transcription of a downstream (3' direction) coding sequence. The promoter sequence is generally bounded at its 3' terminus by the transcription initiation site and extends upstream (5' direction) to include the minimum number of bases or elements necessary to initiate transcription at levels detectable above background. Within the promoter sequence are found a transcription initiation site (conveniently defined for example, by mapping with nuclease SI), as well as protein binding domains (consensus sequences) responsible for the binding of RNA polymerase. The promoter may be operatively associated with other expression control sequences, including enhancer and repressor sequences.

Promoters which may be used to control gene expression include, but are not limited to, cytomegalovirus (CMV) promoter (Patents No. 5,385,839 and No. 5,168,062), the SV40 early promoter region (Benoist et al. (1981), the promoter contained in the 3' long terminal repeat of Rous sarcoma virus (Yamamoto et al. (1980)), the herpes thymidine kinase promoter (Wagner et al. (1981)), the regulatory sequences of the metallothionein gene (Brinster et al. (1982)) ; prokaryotic expression vectors such as the beta-lactamase promoter (Villa-Komaroff et al. (1978)), or the tac promoter (DeBoer et al. (1983) and (1980) "Useful proteins from recombinant bacteria" in Scientific American) ; promoter elements from yeast or other fungi such as the Gal 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells (Mogram et al. (1985) ; Kollias et al. (1986)), hematopoietic stem cell differentiation factor promoters, erythropoietin receptor promoter (Maouche et al. (1991)), etc.

The terms "mutant" and "mutation" mean any detectable change in genetic material, e.g. DNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. The

term "variant" may also be used to indicate a modified or altered gene, DNA sequence, enzyme, cell, etc., i.e., any kind of mutant.

"Sequence-conservative variants" of a polynucleotide sequence are those in which a change of one or more nucleotides in a given codon position results in no alteration in the amino acid encoded at that position.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Amino acids other than those indicated as conserved may differ in a protein or enzyme so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide or enzyme which has at least 60 % amino acid identity as determined by BLAST or FASTA algorithms, preferably at least 75 %, most preferably at least 85%, and even more preferably at least 90 %, and which has the same or substantially similar properties or functions as the native or parent protein or enzyme to which it is compared.

As used herein, the term "homologous" in all its grammatical forms and spelling variations refers to the relationship between proteins that possess a "common evolutionary origin," including proteins from superfamilies (e.g., the immunoglobulin superfamily) and homologous proteins from different species (e.g., myosin light chain, etc.) (Reeck et al., (1987)). Such proteins (and their encoding genes) have sequence homology, as reflected by their sequence similarity, whether in terms of percent similarity or the presence of specific residues or motifs at conserved positions.

Accordingly, the term "sequence similarity" in all its grammatical forms refers to the degree of identity or correspondence between nucleic acid or amino acid sequences of proteins that may or may not share a common evolutionary origin (see Reeck et al., supra). However, in common usage and in the instant application, the term "homologous," when modified with an adverb such as "highly," may refer to sequence similarity and may or may not relate to a common evolutionary origin.

In a specific embodiment, two DNA sequences are "substantially homologous" or "substantially similar" when at least about 80 %, and most preferably at least about 90 or 95 %, of the nucleotides match over the defined length of the DNA sequences, as determined by sequence comparison algorithms, such as BLAST, FASTA, DNA Strider, etc. An example of such a sequence is an allelic or species variant of the specific genes of the invention. Sequences that are substantially homologous can be identified by comparing the sequences using standard software available in sequence data banks, or in a Southern hybridization experiment under, for example, stringent conditions as defined for that particular system.

Similarly, in a particular embodiment, two amino acid sequences are "substantially homologous" or "substantially similar" when greater than 80 % of the amino acids are identical, or greater than about 90 % are similar (functionally identical). Preferably, the similar or homologous sequences are identified by alignment using, for example, the GCG (Genetics Computer Group, Program Manual for the GCG Package, Version 7, Madison, Wisconsin) pileup program, or any of the programs described above (BLAST, FASTA, etc.).

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., supra). The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridization with shorter nucleic acids, i.e., oligonucleotides, the position of mismatches becomes more important, and the length of the oligonucleotide determines its specificity (see Sambrook et al., supra, 11.7-11.8). A minimum length for a hybridizable nucleic acid is at least about 10 nucleotides ; preferably at least about 15 nucleotides ; and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence. Vectors include plasmids, phages, viruses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of doublestranded DNA, usually of bacterial origin, that can readily accept additional (foreign) DNA and which can be readily introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts. Nonlimiting examples include pKK plasmids (Clontech), pUC plasmids, pET plasmids (Novagen, Inc., Madison, WI), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, e.g. antibiotic resistance, and one or more expression cassettes.

The terms "express" and "expression" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, e.g. the resulting protein, may also be said to be "expressed" by the cell. An expression product can be characterized as intracellular, extracellular or secreted. The term "intracellular" means something that is inside a cell. The term "extracellular" means something that is outside a cell. A substance is "secreted" by a cell if it appears in significant measure outside the cell, from somewhere on or inside the cell.

The term "transfection" means the introduction of a foreign nucleic acid into a cell. The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA bas been "transformed" and is a "transformant" or a "clone." The DNA or RNA introduced into a host cell can come from any source, including cells of the same genus or species as the host cell, or cells of a different genus or species.

The term "host cell" means any cell of any organism that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described infra.

The term "expression system" means a host cell and compatible vector under suitable conditions, e.g. for the expression of a protein coded for by foreign DNA carried by the vector and introduced to the host cell. Common expression systems include E. coli host cells and plasmid vectors, insect host cells and Baculovirus vectors, and mammalian host cells and vectors. In a specific embodiment, the protein of interest is expressed in COS-1 or C2C12 cells. Other suitable cells include CHO cells, HeLa cells, 293T (human kidney cells), mouse primary myoblasts, and NIH 3T3 cells.

The therapeutic or diagnostic methods of the invention are useful for any subject or patient. The patient is a human, preferably an adult, but the methods according to the present invention can also be applied to mammals or other vertebrates.

### Therapy

In a first aspect of the invention it is provided a method for preventing demyelination, and/or enhancing axonal myelination, which method comprises modulating the CD100 semaphorin or a receptor thereof.

Another subject of the invention is the use of a modulator of the CD100 semaphorin or of a modulator of a CD100 semaphorin receptor for the preparation of a medicament useful in the prevention of demyelination and/or for enhancement of axonal myelination.

The medicament is particularly useful in the prevention or treatment of a myelin disorder, or a disease involving oligodendrocytes. More particularly such diseases include multiple sclerosis, HTLV1-associated myelopathy, oligodendrogliomas and leucodystrophies. One can also cite Alexander disease, Canavan disease, Krabbe disease, Pelizaeus-Merzbacher disease, Zellweger disease, Refsum disease, CACH disease, X-linked adrenoleucodystrophy, adrenoleucodystrophy, adrenomyeloneuropathy or leucodystrophies of undetermined origin.

In a second aspect of the invention, it is provided a method for stimulating axonal regeneration, which method comprises modulating the CD100 semaphorin or a receptor thereof.

Another subject of the invention is the use of a modulator of the CD100 semaphorin or of a modulator of a CD100 semaphorin receptor for the preparation of a medicament useful to stimulate axonal regeneration.

The medicament is particularly useful in the treatment of central nervous system (CNS) lesions, for example caused by spinal cord injury, neurodegenerative diseases (e.g. Alzheimer's disease or Parkinson's disease), leukodystrophies, or stroke.

The present invention also provides a method for preventing demyelination, and/or enhancing axonal myelination, which method comprises administering to a patient in need of such treatment a therapeutically active amount of a CD 100 modulating agent, or a CD 100 receptor modulating agent.

Another subject of the invention is a method for stimulating axonal regeneration, which method comprises administering to a patient in need of such treatment a therapeutically active amount of a CD100 inhibiting agent, or a CD100 receptor inhibiting agent.

### Gene therapy

In accordance with the present invention, the modulation of CD100 activity may be achieved by augmenting the amount of CD100 protein or of one of its receptors in the cells of a patient. This may be performed by transfecting the cells with a CD100 expressing vector or a CD100 receptor expressing vector, e.g. in the form of a naked DNA or as a viral vector.

Preferably, the CD100 or CD100 receptor nucleic acid forms part of a vector. Such vector is a nucleic acid comprising a CD100 or CD100 receptor coding sequence operatively associated with sequences that control expression of the CD 100 or CD 100 receptor in a cell transfected with the vector.

The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

The vector used may be of diverse origin, as long as it is capable of transforming animal cells, preferably human tumour cells. In a preferred embodiment of the invention, a viral vector is used which can be chosen from adenoviruses, retroviruses, adeno-associated viruses (AAV), lentivirus, herpes virus, cytomegalovirus (CMV), vaccinia virus and the like. Vectors derived from adenoviruses, retroviruses or AAVs, HIV-derived retroviral vectors, incorporating heterologous nucleic acid sequences have been described in the literature (Akli et al., (1993); Stratford-Perricaudet et al. (1990) ; EP 185 573, Levrero et al. (1991) ; Le Gal la Salle et al. (1993) ; Roemer et al. (1992) ; Dobson et al. (1990) ; Chiocca et al. (1990) ; Miyanohara et al. (1992) ; WO 91/18088).

The present invention therefore also relates to any recombinant virus comprising, inserted into its genome, a CD100 or CD100 receptor nucleic acid sequence.

Advantageously, the recombinant virus according to the invention is a defective virus. The term "defective virus" designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the CD100 or CD100 receptor nucleic acid of the invention. Preferably, the defective virus nevertheless conserves the sequences of its genome which are necessary for the encapsulation of the viral particles.

It is particularly advantageous to use the nucleic acid sequences of the invention in a form incorporated in an adenovirus, an AAV or a defective recombinant retrovirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA. Among the various serotypes, the use of the type 2 or 5 adenoviruses (Ad2 or Ad5) is preferred within the framework of the present invention. In the case of the Ad5 adenoviruses, the sequences necessary for the replication are the E1A and E1B regions.

The defective recombinant viruses of the invention can be prepared by homologous recombination between a defective virus and a plasmid carrying, inter alia, the CD100 or CD100 receptor encoding sequence (Levrero et al. (1991) ; Graham, (1984)). The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 (Graham et al. (1977)) which contains especially, integrated into its genome, the left part of the genome of an Ad5 adenovirus (12%). As example of a line which can be used for the preparation of defective recombinant retroviruses, there may be mentioned the CRIP line (Danos et al. (1988)). Alternative vectors, such as shuttle vectors, can also be used that permit the cloning of the desired gene in the vector backbone.

Then the viruses which have multiplied are recovered and purified according to conventional molecular biology techniques.

Targeted gene delivery is described in International Pat. Publication WO 95/28494, published October 1995.

Alternatively, the vector can be introduced in vivo by lipofection. For the past decade, there has been increasing use of liposomes for encapsulation and transfection of nucleic acids in vitro. Information regarding liposome is provided in the "pharmaceutical composition" section of the present application as well. Synthetic cationic lipids designed to limit the difficulties and dangers encountered with liposome mediated transfection can be used to prepare liposomes for in vivo transfection of a gene encoding a marker (Felgner, et. al. (1987) ; Mackey, et al., (1988). The use of cationic lipids may promote encapsulation of negatively charged nucleic acids, and also promote fusion with negatively charged cell membranes (Felgner et al. (1989). The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting (see Mackey, et. al., supra). Targeted peptides, e.g. hormones or neurotransmitters, and proteins such as antibodies, or non-peptide molecules could be coupled to liposomes chemically.

It is also possible to introduce the vector in vivo as a naked DNA plasmid. Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun, or use of a DNA vector transporter (see, e.g., Wu et al., (1992); Wu et al. (1988)).

Such gene therapy may be particularly advantageous to treat a disease caused by a mutant form of CD100 or of a CD100 receptor.

### Peptide therapy

In another embodiment, the augmentation of the amount of CD100 or CD100 receptor protein in the cells of a patient may be achieved by administering the cell or the patient with a CD100 or CD100 receptor protein, or a CD100 or CD100 receptor peptide.

For that purpose, the a CD100 or CD100 receptor protein or peptide may be chemically or enzymatically modified to improve stability or bioavailability.

### Modulating agents

In another embodiment, modulating, i.e. augmenting or lowering the amount or activity of a CD100 or CD100 receptor protein in a cell is achieved by stimulating or inhibiting the CD100 or CD100 receptor endogenous expression or activity in the cell.

One skilled in the art knows how to develop or select useful modulators. Methods for screening CD100 modulators are described in the international patent application WO 97/17368.

Modulating agents can be identified by screening methods, including biochemical and cellular *in vitro* assays.

Of particular interest is a process for screening substances for their ability to modulate, *i.e.* inhibit or activate, CD100 or CD100 receptor expression or activity, comprising the steps of providing a cell that expresses CD100 or CD100 receptor and testing the ability of the substances to modulate, *i.e.* inhibit or activate, CD100 or CD100 receptor expression or activity, e.g. by blocking the interaction between CD100 and one of its receptor, or binding partners. The enhancement of level of expression or activity of the CD100 or CD100 receptor in comparison with a CD100 or CD100 receptor expressing cell that was not subjected to this agent, is indicative of an agent that shows a stimulating property toward the CD100 or CD100 receptor.

Test substances can be of any type, including natural or synthetic compounds or mixtures of compounds. The substance may be structurally defined or of unknown structure, *e.g.* in the form of a biological extract.

Preferred modulator encompass any agent that activates CD100 or CD100 receptor activity through direct interaction with the CD100 protein or CD100 receptor protein. Alternatively one may employ any agent that inhibits CD100 or CD100 receptor activity through direct interaction with the CD100 protein or CD100 receptor protein.

Suitable CD100 modulators encompass inhibitory forms of CD100, which can be modified forms of CD100 molecules, fragments of CD100 molecules, or modified fragments. Fragments comprising the semaphorin domain (aa 42-aa 554), and/or the immunoglobulin domain (aa565-aa629) are preferred.

CD100 molecules or fragments thereof that retain binding capacity to CD100 receptors can then be mutated, such as by site directed mutagenesis for obtaining a form of CD100 or fragment thereof that retains binding capacity but fails to deliver a CD100 ligand-associated signal. Other useful modulators comprise or consist in an extracellular fragment of a CD100 semaphorin receptor. Fragments that comprise the Semaphorin domain, the plexin repeats, and/or the IPT/TIG repeats are preferred.

Proteases that cut or inactivate CD100 or CD100 receptors may also be useful. In that respect inhibitors of metalloproteases previously used to inhibits CD100 cleavage (as described in Elhabazi et al., 2001) may be of interest.

Preferred CD100 modulators include inhibitory anti-CD100 antibodies.

### Inhibitory antibodies

The term "antibody" in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')2 and F(v).

Antibodies that inhibit the interaction of CD100 semaphorin with its receptor are more particularly useful.

Whereas polyclonal antibodies may be used, monoclonal antibodies are preferred for they are more reproducible in the long run.

Procedures for raising polyclonal antibodies are also well known. Typically, such antibodies can be raised by administering the protein or polypeptide of the present invention subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in E. Harlow, et. al., editors, Antibodies: A Laboratory Manual (1988), which is hereby incorporated by reference.

A "monoclonal antibody" in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., Antibodies: A Laboratory Manual, Cold Spring Harbor Laboratory, New York, (1988)). Monoclonal antibodies (Mabs) may be prepared by immunizing purified CD100 protein isolated from any of a variety of mammalian species into a mammal, e.g. a mouse, rat, rabbit, goat, human and the like mammal. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

While Mabs can be produced by hybridoma culture, the invention is not to be so limited. Also contemplated is the use of Mabs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No. WO 890099 to Robinson et al.; European Patent Publications No. 0239400 to Winter et al. and No. 0125023 to Cabilly et al. In addition, the literature provides methods for forming chimeric antibodies, humanized antibodies, single chain antibodies and the like variations on a basic immunoreactive antibody fragment. All of these are considered within the scope of the invention insofar as a class and specificity of antibody is disclosed and claimed, regardless of the precise variant structure that one skilled in the art may construct.

Several antibodies are already on the market. Anti-plexin-B1 antibodies can be purchased from Santa Cruz Biotech (Ca, USA). Several anti-CD72 antibodies are also commercially available (Pharmingen; Immunotech). CD100 antibodies can be purchased from Transduction laboratories, KY, USA or Santa Cruz Biotech (CA, USA).

### Inhibition of CD100 transcription or CD100 receptor transcription

According to one embodiment of the invention, inhibition of CD100 activity is achieved by inhibiting the transcription of the CD100 semaphorin or of its receptor.

One skilled in the art can select various strategies therefor.

For instance, antisense nucleic acids, including ribozymes, may be used to inhibit expression of CD 100 protein or one of its receptors.

When transfected into a cell via a viral vector antisense nucleic acids can be stably integrated and provide a long-term inhibition. Alternatively, antisense oligonucleotides can be directed administered and provides a transient inhibition.

An "*antisense nucleic acid*" is a single stranded nucleic acid molecule which, on hybridizing under cytoplasmic conditions with complementary bases in an RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes and RNase-H mediated arrest. Antisense nucleic acid molecules can be encoded by a recombinant gene for expression in a cell (*e.g.,* U.S. Patent No. 5,814,500 ; U.S. Patent No. 5,811,234), or alternatively they can be prepared synthetically (*e.g.,* U.S. Patent No. 5,780,607).

"*CD100 antisense"* nucleic acids are preferably designed to be specifically hybridizable with a CD100 encoding sequence, e.g. the human sequence.

Antisense therapy usually makes use of a vector (e.g. a plasmid) that carries the antisense sequence.

The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

Vectors and methods for administering the same may be selected from the vectors and methods described for gene therapy, or may be adapted therefrom.

Antisense oligonucleotides can also be used to provide a transient CD100 inhibition. For that purpose antisense, oligonucleotides, that are not part of a viral vector, can be administered to the cell by any means as described above.

As used herein, the term "oligonucleotide" refers to a nucleic acid, generally of at least 10, preferably at least 15, and more preferably at least 20 nucleotides, preferably no more than 100 nucleotides, that is hybridizable to a genomic DNA molecule, a cDNA molecule, or a mRNA molecule encoding a gene, mRNA, cDNA, or other nucleic acid of interest.

Oligonucleotides can be labelled, *e.g.,* with ³²P-nucleotides or nucleotides to which a label, such as biotin, has been covalently conjugated. Generally, oligonucleotides are prepared synthetically, preferably on a nucleic acid synthesizer. Accordingly, oligonucleotides can be prepared with non-naturally occurring phosphoester analog bonds, such as thioester bonds, etc.

Reversible short inhibition of transcription may also be useful as described above. Such inhibition can be achieved by specific inhibitors of CD100 transcription.

### Pharmaceutical compositions

CD100 modulators can be formulated in pharmaceutical compositions, for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected, optionally directly into the nervous system or in the brain, *e.g.* by intracerebroventricular injection for dispersion into other areas.

Antibodies against CD100/sema4D or their receptors, in particular type B plexins and CD72, CD100 or CD100 receptor peptides, or recombinant proteins may also be injected into the nervous system to stimulate the proliferation, the differentiation, the migration or the survival of oligodendrocytes, for instance to stimulate remyelination in pathological conditions or stop the demyelination. These reagents may particularly be used to interfere with the abnormal development of oligodendrocytes in pathological conditions such as oligodendrogliomas.

The suitable pharmaceutical compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses of CD100 or CD100 receptor modulator used for the administration can be adapted as a function of various parameters, and in particular as a function of the mode of administration used, of the relevant pathology, or alternatively of the desired duration of treatment.

To prepare pharmaceutical compositions for peptide or antibody therapy, an effective amount of the protein may be dissolved or dispersed in a pharmaceutically acceptable carrier or aqueous medium.

Examples of pharmaceutical formulations are provided hereafter.

Pharmaceutical compositions comprise an effective amount of a CD100 modulating agent in a pharmaceutically acceptable carrier or aqueous medium.

"Pharmaceutically" or "pharmaceutically acceptable" refer to molecular entities and compositions that do not produce an adverse, allergic or other untoward reaction when administered to an animal, or a human, as appropriate.

As used herein, a "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The pharmaceutical forms suitable for injectable use include sterile aqueous solutions or dispersions ; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using peptide therapeutics as active ingredients, U.S. patents 4,608,251 ; 4,601,903 ; 4,599,231 ; 4,599,230 ; 4,596,792 and 4,572,770 provide useful information.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The CD100 modulating agents or the CD100 receptor modulating agents may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration ; liposomal formulations ; time release capsules ; and any other form currently used, including creams.

Other routes of administration are contemplated, including nasal solutions or sprays, aerosols or inhalants, or vaginal or rectal suppositories and pessaries.

In certain embodiments, the use of liposomes and/or nanoparticles is contemplated for the introduction of inhibitory antibodies or other agents, especially protein or peptide agents, as well as nucleic acid vectors into host cells. The formation and use of liposomes is generally known to those of skill in the art, and is also described below.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 A, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs as a characteristic phase-transition temperature and results in an increase in permeability to ions, sugars and drugs.

Liposomes interact with cells via four different mechanisms: endocytosis by phagocytic cells of the reticuloendothelial system such as macrophages and neutrophils ; adsorption to the cell surface, either by non-specific weak hydrophobic or electrostatic forces, or by specific interactions with cell-surface components ; fusion with the plasma cell membrane by insertion of the lipid bilayer of the liposome into the plasma membrane, with simultaneous release of liposomal contents into the cytoplasm ; and by transfer of liposomal lipids to cellular or subcellular membrane, or vice versa, without any association of the liposome contents. Varying the liposome formulation can alter which mechanism is operative, although more than one may operate at the same time.

### Diagnosis

The present invention further relates to an *in vitro* method for diagnosis or determination of the evolution of a condition involving a demyelination, which method comprises detecting in a biological sample of a test subject, the expression of a CD100 semaphorin or of a receptor thereof, compared with the expression of a CD100 semaphorin or of a receptor thereof in a biological sample of a control subject, wherein an increased expression of CD100 or of a receptor thereof is indicative of said condition or of a poor prognosis.

A "biological sample" is a fluid from a subject, including serum, blood, spinal fluid, cerebrospinal fluid, urine, or a tissue extract or a tissue or organ biopsy such as brain, spinal cord or nerves.

"A subject" or "a patient" is a vertebrate, *e.g.* a mammal, preferably a human being, regardless of his/her age, sex and general condition. Children and infants are also encompassed. The test subject may be asymptomatic, may be considered likely to develop the disease or condition. Subjects with a suspicion of myelin disorder or subjects who have already shown symptoms of the disease or condition can also be tested. Subjects who are predisposed to developing a myelin disorder naturally are a preferred target.

The «control subject» may be a healthy subject or a subject without any myelin disorder. In order to determine the evolution of a condition involving a demyelination, it may be very useful to test a subject for the expression of CD100 or of a receptor thereof, and to monitor the effect of a drug or the spreading of the condition, by testing him/her a second time, *e.g.* a few weeks later. In that case the results of the second test are compared with the results of the first test, and in general also with the results obtained with a "healthy" subject. The "control subject" then refers either to the same test subject or to a "healthy subject".

"A condition involving a demyelination" includes any myelin disorder, as described above.

The term "diagnosis" refers to the determination or the confirmation of a disease or condition in a subject. The term "poor prognosis" means that the condition has worsened.

The "expression of a CD100 semaphorin or of a receptor thereof" may be determined either by assaying the CD100 semaphorin protein or a receptor thereof, or by determining the level of transcription, *i.e.* the quantity of mRNA that encodes CD 100 semaphorin or a receptor thereof.

The "activity of a CD100 semaphorin or of a receptor thereof" refers to the binding ability of the CD100 semaphorin toward one of its receptors, and to the activation of these receptors. This may be determined by standard pharmacological tests. For instance one may perform binding assays with a iodinated- or tritiated CD100, or with CD100-fused to human placental alkaline phosphatase as described in Tamagnone et al., 1999.

Various methods for detecting and/or quantifying the expression of CD100 semaphorin protein or a receptor thereof are described in greater detail below.

Alternatively the presence of anti-CD100 auto-antibodies may be determined by means of CD100 proteins or epitopic fragments thereof, and the immune complexes formed between said proteins and said antibodies present in a biological sample are detected by standard methods.

Methods for assaying the CD100 semaphorin protein or a receptor thereof are well known by one skilled in the art.

Such methods comprise contacting a biological sample with a binding partner capable of selectively interacting with a CD100 molecule present in the sample. The binding partner is generally an antibody, that may be polyclonal or monoclonal, preferably monoclonal.

Methods for producing antibodies as described above in accordance with therapy can also be easily adapted to produce antibodies useful for the diagnostic methods according to the invention.

For example, the presence of CD100, or a mutated form of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound CD100 in a liquid phase from a solid phase support to which antigen-antibody complexes are bound. Solid supports which can be used in the practice of the invention include substrates such as nitrocellulose (e.g., in membrane or microtiter well form); polyvinylchloride (*e.g.,* sheets or microtiter wells); polystyrene latex (e.g., beads or microtiter plates); polyvinylidine fluoride; diazotized paper; nylon membranes; activated beads, magnetically responsive beads, and the like.

Typically, a solid support is first reacted with a solid phase component (*e.g.,* one or more anti-CD100 antibody) under suitable binding conditions such that the component is sufficiently immobilized to the support according to methods well known to those skilled in the art. After reacting the solid support with the solid phase component, any non-immobilized solid-phase components are removed from the support by washing, and the support-bound component is then contacted with a biological sample suspected of containing ligand moieties (e.g., CD100 molecules toward the immobilized antibodies) under suitable binding conditions. After washing to remove any non-bound ligand, a secondary binder moiety is added under suitable binding conditions, wherein the secondary binder is capable of associating selectively with the bound ligand. The presence of the secondary binder can then be detected using techniques well known in the art.

More particularly, an ELISA method can be used, wherein the wells of a microtiter plate are coated with an anti-CD 100 antibody. A biological sample containing or suspected of containing CD100 molecules is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample CD100 molecules, the plate washed and the presence of the secondary binding molecule detected using methods well known in the art.

Thus, in one particular embodiment, the presence of bound CD100 molecules from a biological sample can be readily detected using a secondary binder comprising another antibody, that can be readily conjugated to a detectable enzyme label, such as horseradish peroxidase, alkaline phosphatase or urease, using methods known to those of skill in the art. An appropriate enzyme substrate is then used to generate a detectable signal. In other related embodiments, competitive-type ELISA techniques can be practiced using methods known to those skilled in the art.

Assays can also be conducted in solution, such that the antigenic polypeptides and antibodies specific for those proteins form complexes under precipitating conditions. In one particular embodiment, antibodies can be attached to a solid phase particle (e.g., an agarose bead or the like) using coupling techniques known in the art, such as by direct chemical or indirect coupling. The antibody-coated particle is then contacted under suitable binding conditions with a biological sample suspected of containing CD100 molecules. The particle-antigen-antibody complexes aggregate and can be precipitated and separated from the sample using washing and/or centrifugation. The reaction mixture can be analyzed to determine the presence or absence of antibody-antigen complexes using any of a number of standard methods, such as those immunodiagnostic methods described above.

The above-described assay reagents, including the anti-CD100 antibodies, can be provided in kits, with suitable instructions and other necessary reagents, in order to conduct immunoassays as described above. The kit can also contain, depending on the particular immunoassay used, suitable labels and other packaged reagents and materials (*i.e.* wash buffers and the like). Standard immunoassays, such as those described above, can be conducted using these kits.

Alternatively, auto-antibodies directed against CD 100 molecules can be detected by using essentially the same type of assays, *e.g.* by immobilising a CD100 molecule or epitopic fragment thereof on a solid support.

Methods for determining the level of transcription of a gene are also well known in the art. For example the nucleic acid contained in the samples (*e.g.,* cell or tissue prepared from the subject) is first extracted according to standard methods, for example using lytic enzymes or chemical solutions or extracted by nucleic-acid-binding resins following the manufacturer's instructions. The extracted mRNA is then detected by hybridization (*e.g.,* Northern blot analysis) and/or amplification (*e*.*g*., PCR). Nucleic acids having at least 10 nucleotides, preferably between about 20 and 25 nucleotides, even more preferably between about 50 and 100 nucleotides, and exhibiting sequence complementarity or homology to the CD100 mRNA herein find utility as hybridization probes. It is understood that probes need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e.g., avidin/biotin). Further, the nucleic acids can also be attached to a solid support (e.g., glass or chip) for use in high throughput screening assays using methods described, for example, in U.S. Patents. Nos. 5,405,783, 5,578,832 and 5,445,934. Results of high throughput assays can be analyzed using computer software programs available from the manufacturers.

### CD100-/- oligodendrocytes

In another aspect of the invention, the CD100 gene is inactived in oligodendrocytes or precursor cells thereof, to produce CD100-/- cells particularly useful for axonal remyelination.

These are human or non-human mammalian oligodendrocytes or precursor (or progenitor) cells thereof, *e.g.* from mouse or other rodents, or other mammals in particular primates.

"Oligodendrocyte precursor cells" are cells that can be isolated from embryonic or adult brain, and can develop *in vitro* and *in vivo* into various type of glial cells, including oligodendrocytes. Different types of precursors with various differentiation potential can be characterized using antibody markers such as NG2 chondroitin sulfate, 04 glycolipid, the transcription factors olig1 and olig2, and the platelet-derived growth factor alpha receptor (see Levine et al, 2001).

Inactivation of the CD100 gene may be achieved by homologous recombination in ES (embryonic stem) cells, for example as described in Shi et al., Immunity. 2000 Nov;13(5):633-42.

Another subject of the present invention is thus a pharmaceutical composition comprising CD100-/- oligodendrocytes or precursor cells thereof, wherein CD100 gene is inactived, in association with a pharmaceutically acceptable carrier.

One skilled in the art may readily prepare such pharmaceutical compositions, by following the guidance provided above. It is desired that the composition be suitable for grafting into the brain, more particularly in damaged regions of, preferably by direct injection, e.g. following procedures reviewed in Nait-Oumesmar et al., 2000.

The present invention contemplates the use of CD100-/- oligodendrocytes or precursor cells thereof, wherein CD100 gene is inactived, for the preparation of a medicament useful for axonal remyelination.

A further subject of the invention is thus a method for enhancing axonal remyelination, which method comprises administering, *e.g.* grafting, to a subject in need thereof an efficient amount of CD100 -/- oligodendrocytes or precursor cells thereof. This method is particularly advantageous for patients who suffer from a myelin disorder such as multiple sclerosis.

### Purification of oligodendrocytes

CD100 antibodies, peptides or recombinant proteins may also be used to purify dissociated oligodendrocytes by affinity, for instance using immuno-panning, or magnetic beads. This is particularly useful for *in vitro* cultures of oligodendrocytes or to compare gene or protein expression pattern between normal or "abnormal" oligodendrocytes in a screening method. Likewise antibodies against CD100 receptor, in particular plexin B, or recombinant CD100 receptor proteins or peptide can be used to purify oligodendrocytes.

### Animal models

Several animal models have been developed to analyze the mechanisms of demyelination and remyelination (Nait-Oumesmar et al., 2000), for instance the intracerebral injection of lysolecithin (LPC) can induce a local demyelination around the injection site. However an extensive demyelination can be obtained by immunization of specific mouse strains with brain homogeneates or myelin proteins such as MOG (Linington et al, 1992). These treatments induce an auto-immune disease called experimental allergic encephalomyelitis (EAE). These models are very useful tools to study the mechanisms and the molecules that are involved in remyelination and could help understanding the process of multiple sclerosis and develop novel treatments.

The invention provides an alternative method for producing a demyelination in an animal model. This method could be more efficient (and lower cost, ) to induce demyelination, and the symptoms developed in treated animals closer to known myelin pathologies. Another purpose is to experimentally induce demyelinated lesions in animals to develop models of leucodystrophy. A subject of the invention is thus a method for inducing a demyelination in a non-human animal model, which method comprises administering to the animal model a CD100 semaphorin, a biologically active fragment thereof, or a receptor thereof such as type B plexins.

Such animal models include, without limitation, rodents, especially mouse, rat, rabbit, or other mammals.

"Biologically active fragments" refer to fragments of the CD100 semaphorin that retain the ability of the CD100 semaphorin to bind and activate at least one of its receptors, and/or to induce or enhance demyelination in the animal.

The CD100 semaphorin or a biologically active fragment thereof may be administered to the animal by any suitable route, e.g. by injection in the blood system of the animal.

As a consequence the animal develops a demyelination, that provides an interesting model for multiple sclerosis.

The examples illustrate the invention without restricting its scope.

### EXAMPLES:

### Example 1: CD100 and CD100 receptors expression in oligodendrocytes

### 1.1 Expression of CD100 in oligodendrocytes in vivo

The inventors first used *in situ* hybridization to study CD100/Sema4D expression in the developing and adult rat and mouse brain. Wistar rats, OF1 and C57/B16 mice (IFFA-Credo, Lyon, France) were used for *in situ* hybridization and immunocytochemical studies. The day on which a vaginal plug was detected was considered embryonic day 0 (E0), and the day of birth postnatal day 0 (P0). Animals were anesthetized with chloral hydrate (350 mg/kg, i.p).

Animals were perfused transcardially with 4% paraformaldehyde in 0,1 M phosphate buffer, pH 7.4 (PFA), their brains were dissected and post-fixed overnight in 4% PFA. They were then cryoprotected in 10% sucrose, frozen in isopentane -55°C and stored at -80°C until sectioning. Serial coronal, horizontal and sagittal sections (25µm) were cut with a Cryostat (Leica) and stored at -80°C.

Antisense riboprobes were labelled using the Riboprobe synthesis kit (Promega) with digoxigenin-11-d-UTP (Roche Diagnostics) or ³⁵S-UTP (> 1000 Ci/mM; Amersham) by *in vitro* transcription of cDNAs encoding mouse CD100 (Furuyama et al., 1997).

For non-radioactive *in situ* hybridization, tissue sections were fixed for 10 min in 4% PFA, washed in PBS (phosphate buffer saline), treated with proteinase K (10 µg/ml; Eurobio) for 7 min 30 sec, post-fixed for 5 min in 4% PFA, washed in PBS, acetylated, washed in PBS 1% Triton X-100. Slides were incubated 2 hours at room temperature with hybridization buffer (50% formamide, 5X SSC, 1X denhardt's, 250 µg/ml yeast tRNA and 500 µg/ml herring sperm). Then, tissue sections were hybridized overnight at 72°C with riboprobes (1/200). Following hybridization, sections were rinsed for 2 hours in 2X SSC at 72°C, and blocked in blocking reagent (B1: 0.1 M Tris pH7.5, 0.15 M NaCl, 10% normal goat serum (NGS)) for 1 hour at room temperature. After blocking, slides were incubated overnight at room temperature with anti-DIG antibody conjugated with the alkaline phosphatase (1/5000, Roche Diagnostics) in B1 buffer containing 1% NGS. After washing in B1 buffer, the alkaline phosphatase activity was detected using nitroblue tetrazolium chloride (337.5 µg/ml) and 5-Bromo-4-chloro-3-indolyl phosphate (175 µg/ml) (Roche Diagnostics).

For *in situ* hybridization with ³⁵S-labeled riboprobes, brain sections were hybridized with ³⁵S-labeled riboprobes. Sections were post-fixed for 15 min in 4% PFA, washed in PBS, acetylated, washed in PBS, dehydrated in graded ethanols and air-dried. Sections were covered with hybridization buffer containing 5 10⁵ cpm/µl of riboprobes (50% formamide, 0.3 M NaCl, 20 mM Tris-HCl pH 7.4, 5 mM EDTA, 1X Denhardt's, 10X dextran sulfate, 10 mM DTT, 10 mM NaH₂PO₄ pH8 and 250 µg/ml yeast tRNA). Slides were hybridized overnight at 48°C in humid chamber. Following hybridization, sections were rinsed for 30 min in 5X SSC at 42°C and for 20 min in 2.5X SCC at 60°C. Sections were then treated for 30 min with 20 mg/ml Rnase A at 37°C, washed in 2X SSC and in 0,1X SSC for 15 min each, dehydrated and air-dried. Autoradiograms were obtained by apposing the sections to hyperfilms (βmax, Amersham) for 3 days. Autoradiographic films were developed in D19 (Kodak) for 4 minutes at room temperature and fixed in AL4 (Ilford) for 4 minutes. For histological analyses, the slides were dipped in photographic emulsion (NTB2, Kodak), exposed for about 12 days and developed.

In order to identify cells expressing CD100 mRNA, in situ hybridization is combined with immunocytochemistry for markers of astrocytes and oligodendrocytes. A rat monoclonal antibody against CD100 (Kumanogoh et al. 2000) is also used as well as a rat monoclonal antibody against PLP (clone AA3), and rabbit polyclonal antibody against glial fibrillary acidic protein (GFAP; Dako), and a mouse monoclonal antibody against myelin basic protein (MBP; Roche Diagnostics).

This study has shown that from birth, CD100 mRNA is only expressed by oligodendrocytes. CD100 expressing cells also express PLP but do not expressed GFAP. CD100 expression is not detected in oligodendrocyte precursors in the embryo. The onset of CD100 expression appears to coincide with the beginning of myelination, as it correlates well with the postnatal appearance of PLP immunoreactivity in oligodendrocytes. CD100 expression was not detected in Schwann cells and increased in oligodendrocytes until one month of age. The level of expression in oligodendrocytes is lower in older animals. An immunocytochemical study using a rat-anti-mouse CD100 monoclonal antibody has confirmed that in the postnatal mouse brain, CD100 is expressed on the cell bodies of myelinating oligodendrocytes and on myelin. CD100 protein was also detected from birth, throughout the spinal cord and brain. CD100 expression peaked at P30, coinciding at that stage with MBP staining. At later stages, CD100 positive oligodendrocytes were still observed but at a lower number, suggesting that CD100 expression was partially downregulated.

### 1.2 Expression of CD100 in oligodendrocytes in vitro

An *in vitro* model of neuron/oligodendrocyte cocultures that recapitulates most steps of myelination that are observed *in vivo* has been developed (Charles et al. 2000). CD100 expression by oligodendrocytes is studied in this system and correlated with the expression of other known markers, such as PLP, A2B5, NG2 and 04. It was found that from 8 days of culture, C100 expressing oligodendrocytes can be detected in the culture. After three weeks of culture, myelin is strongly CD100 immunoreactive.

### 1.3 Expression of CD100 receptor, CD72 and type B plexins in the CNS.

CD100 has two known receptors, plexin-B1 and CD72. The inventors have studied the expression of these molecules in the developing and adult mouse brain to determine if their expression is correlated with CD100 expression. They used radioactive and non-radioactive *in situ* hybridization as described above. Riboprobes were generated with cDNAs encoding mouse CD72 (Ying et al. (1995) mouse plexin B1, plexin B2 and plexin B3 (Cheng et al. (2001). It was found that plexin B1 mRNA is expressed in regions of cell proliferation in the developing and adult brain, such as ventricular zones in embryos, and the subventricular zone in adults. It was also found that in the central nervous system of postnatal mice, plexin B3 mRNA is exclusively expressed by oligodendrocytes. CD72 mRNA was detected from birth in all neurons.

### Example 2 : Role of CD100 in myelination and remyelination

### 2.1. In vitro study of CD100 function in myelination

The role of CD100 in myelination is investigated *in vitro,* using the culture system developed by the Lubetzki group (Charles et al., 2000). Neuron-oligodendrocyte cocultures from 15-day-old mouse fetuses forebrain are prepared and maintained for 11-18 days *in vitro.* First, purified CD100-Fc ectodomain or CD100 antibodies are added to the cultures at different stages and the effect of these treatments on myelination determined using several antibodies (MAP2, Tuj-1 and MBP; see Charles et al., 2000). Similarly CD100 function is perturbed by adding to the cultures purified plexin-B1 ectodomains (Tamagnone et al., 1999). Second, oligodendrocyte/neuron cocultures are performed using embryos from CD100 -/- mice and their development compared to control cultures.

### 2.2. Role of CD100 in demyelination and remyelination in vivo.

To study CD100 possible function in demyelination and remyelination the authors induce EAE in CD100 KO mice using MOG peptide+adjuvant injections. The extent of the demyelination and the subsequent remyelination in the mutant is compared with control wild-type and heterozygous animals from the same background. Classical staining such as Luxol and several antibodies such as MBP, PLP, NG2 and 04 is used in combination with electron microscopy to study lesioned animals. Alternatively, lysolecithin (Decker et al, 2002) is injected unilaterally into the corpus callosum of CD100 knock-out and wild type mice using appropriate stereotaxic coordinates, with a Hamilton syringe containing 2 µl of a solution of 1% LPC (Sigma, St.Louis, MO) in 0.9% NaCl. The injection site is labeled with charcoal dust. The needle is kept in place for 5 min to reduce reflux up the needle track. Incisions are ligated with Vetbond thread. Controls are injected with saline only. The rate and extent of remyelination is compared between control and LPC treated animals. Last, demyelination is performed with cuprizone : Male mice, 6-8 weeks old, are fed with a diet of milled Purina mouse chow containing 0.2% cuprizone (Sigma, St. Louis, Missouri) for up to 6 weeks. (Arnett et al. 2001), and the amount of remyelination between CD100 knock-out and C57/B16 wild-type mice is compared.

### Example 3: CD100 expression changes after brain and spinal cord lesions.

### 3.1. Analysis of the expression of CD100/Sema4D and its receptors in the lesioned brain

The membranes associated with myelinated axons can be divided into at least two types: compact myelin and periaxonal myelin. This latest is specifically enriched with proteins such as CNP and MAG, required for the maintenance of interactions between axonal and oligodendroglial membranes (Schachner and Bartsch, 2000). The precise subcellular localization of CD100 in myelin is studied using electron microscopy and immunogold labelling.

Transections of the corticospinal tract in the dorsal column of the spinal cord are performed on adult mice (Hauben et al. 2001). Likewise, lesions of the cerebellum are performed as previously described (Dusart et al. 1994). Animals are perfused with 4% PFA (as described in example 1) at variable time (from 3 days to 3 months) following these lesions and their spinal cord processed for CD100 in situ hybridization and immunocytochemistry. This allows to determine whether CD100 expression is changed following CNS lesions. The authors found that one week after the lesion, CD100 expression is highly upregulated at the lesion site in the spinal cord and cerebellum. In the cerebellum, many CD100 immunoreactive cells, most likely microglial cells, are detected at the lesion site. In addition, the expression of CD100 is increased in oligodendrocytes in the lesionned cerebellar lobules. In the spinal cord, there is also a strong increase in the number of CD100 immunopositive oligodendrocytes at the lesion site, both on the proximal and distal part of the lesion. One month after the lesion, the level of CD100 expression is still higher than in control animal for cerebellar lesion, but is almost back to control levels for spinal cord lesion. Possible changes in CD100 receptors expression (CD72 and type B plexins) after lesion is studied with riboprobes and antibodies.

### 3.2. In vitro analysis of CD100 axon outgrowth inhibitory activity

So far, all semaphorins tested have been shown to inhibit the elongation of several classes of axons (Raper, 2000), but no data are available for CD100. The inventors performed *in vitro* assays to evaluate the potential inhibitory activity of CD100. A CHO cell line expressing recombinant CD100-extracellular domain fused to human IgG1 (CD100-Fc) or fused to placental human alkaline phosphatase (CD100-AP ; Tamagnone et al., 1999) is used. CD100-Fc can be purified using agarose protein-A. The inhibitory activity of CD100-Fc is tested using bioassays that are used for other myelin inhibitors such as NogoA (Chen et al., 2000; GrandPré et al., 2000). 3T3, PC12 or neurons (postnatal DRG neurons or cerebellar granule cells) are seeded in tissue culture wells coated with purified CD100-Fc. The percentage of spreaded cells (3T3 or PC12) or the amount of neurite outgrowth are measured and compared to control proteins such as NogoA, Sema4B-Fc, or 8D6-Fc. Preliminary data showed that CD100 is a strong inhibitor of 3T3 cell spreading in a dose-dependent manner (90% of the 3T3 cells do not spread on CD100 coated wells). Likewise, neurons are seeded on a CD100-Fc substrate or on CHO cells expressing the full length form of CD100. Finally, as CD100 also exists as a soluble form generated by proteolytic cleavage, the repulsive activity of CD100-Fc released by CHO cell aggregates is tested in repulsion assay in collagen gel using DRG explants and cerebellar granule cell aggregates.

### REFERENCES

Akli et al. (1993) Nature Genetics 3:224
Arnett et al. (2001) Nat Neurosci. 4:1116-22
Ausubel *et al.* eds (1994) *Current Protocols in Molecular Biology,* John Wiley and Sons, Inc.
Benoist et al. (1981) Nature,290:304-310
Brinster et al. (1982) Nature, 296:39-42
Charles et al. (2000) Proc. Natl. Acad. Sci. USA 97:7585-7590
Chen et al. (2000) Nature, 403:434-439
Cheng et al. (2001) Neuron. 32:249-63
Chiocca et al. (1990) New Biol. 2:739
Danos et al. (1988) PNAS 85:6460
DeBoer et al. (1980) "Useful proteins from recombinant bacteria" in Scientific American, 242:74-94
DeBoer et al. (1983) Proc. Natl. Acad. Sci. USA, 80:21-25
Decker et al. (2002) Mol Cell Neurosci. 19:225-38
Delaire et al. (1998) Cell Mol Life Sci. 54:1265-76
Dobson et al. (1990) Neuron 5:353
Dusart et al. (1994) Neuroscience., 63:351-6
Elhabazi et al., (2001) J Immunol.166(7):4341-7
Felgner et al. (1989) Science 337:387-388
Felgner, et. al. (1987) Proc. Natl. Acad. Sci. U.S.A. 84:7413-7417
Freshney eds (1986) *Animal Cell Culture*
Fujisawa et al. (1998) Curr. Opin. Neurobiol. 8:587-592
Furuyama et al. (1997) J Biol Chem. 271:33376-33381
Graham (1984) EMBO J. 3(122917)
Graham et al. (1977) J. Gen. Virol. 36: 59
GrandPre et al (2000) Nature, 403:439-444
Hall et al. (1996) Proc. Natl Acad. Sci. USA 93:11780-11785
Hames et al. (1984) *Transcription and Translation*
Hauben et al. (2001) Proc Natl Acad Sci U S A., 98:15173-8
Hérold et al. (1995) Int Immunol. 7:1-8
Kollias et al. (1986) Cell, 46: 89-94
Kumanogoh et al. (2000) Immunity. 2000, 13:621-31
Le Gal la Salle et al. (1993) Science 259:988
Levine et al (2001) Trends Neurosci. 24:39-47
Levrero et al., (1991) Gene 101:195
Linington et al, (1992) J Neuroimmunol. 40(2-3):219-24
Lubetzki et al. (1993) Proc. Natl. Acad. Sci. USA 90:6820-6824
Mackey, et al., (1988) Proc. Natl. Acad. Sci. U.S.A. 85:8027-8031
Maestrini et al. (1996) Proc. Natl. Acad. Sci. USA 93:674-678
Maouche et al. (1991) Blood, 15:2557
Merkler et al. (2001) J. Neurosci. 21:3665-3673
Miyanohara et al., (1992) New Biol. 4:238
Mogram et al. (1985) Nature, 315:338-340
Nait-Oumesmar et al. (2000) Pathol. Biol. 48:70-79
Perbal, (1984) *A Practical Guide To Molecular Cloning*
Raper J. (2000) Curr. Opin. Neurobiol. 10 : 88-94
Reeck et al. (1987) Cell 50:667
Roemer et al. (1992) Eur. J. Biochem. 208-211
Sambrook et al., (1989) *Molecular Cloning: A Laboratory Manual,* Second Edition Cold Spring Harbor Laboratory Press, Cold Spring Harbor, New York
Schachner et al. (2000) Glia, 29:154-65
Shi et al. (2000), Immunity. 13(5):633-42.
Stratford-Perricaudet et al. (1990) Human Gene Therapy 1:241
Tamagnone et al. (1999) Cell 99:71-80
Tamagnone and Comoglio (2000) Trends Cell Biol. 10:377-383
Vikis HG, Li W, Guan KL (2002). Genes Dev. 16(7):836-45
Villa-Komaroff et al. (1978) Proc. Natl. Acad. Sci. USA,75:3727-3731
Wagner et al. (1981) Proc. Natl. Acad. Sci. USA, 78: 1441-1445
Wu et al. (1988) J. Biol. Chem. 263:14621-14624
Wu et al., (1992) J. Biol. Chem. 267:963-967
Yamamoto et al. (1980) Cell, 22:787-797
Ying et al. (1995) Immunol., 154:2743-52

## Claims

1. Use of a modulator of the CD100 semaphorin expression or activity or of a modulator of a CD100 semaphorin receptor expression or activity for the preparation of a medicament useful in the prevention of demyelination and/or for enhancement of axonal myelination.

2. The use according to claim 1, wherein the medicament is useful in the prevention or treatment of a myelin disorder.

3. The use according to claim 2, wherein the myelin disorder is multiple sclerosis.

4. The use according to claim 2, wherein the myelin disorder is leucodystrophy.

5. Use of a modulator of the CD100 semaphorin or of a modulator of a CD100 semaphorin receptor for the preparation of a medicament useful to stimulate axonal regeneration.

6. The use according to claim 5, wherein the medicament is useful to treat central nervous system (CNS) lesions.

7. The use according to any of claims 1 to 6, wherein the modulator is an agent that inhibits CD100 or CD100 receptor activity through direct interaction with the CD 100 protein or CD100 receptor protein.

8. The use according to claim 7, wherein the modulator is an anti-CD100 antibody.

9. The use according to any of claims 1 to 6, wherein the modulator is an extracellular fragment of a CD100 semaphorin receptor.

10. The use according to claim 9, wherein the CD100 semaphorin receptor is CD72 or a plexin B.

11. The use according to any of claims 1 to 6, wherein the modulator is a CD100 or CD100 receptor antisense nucleic acid.

12. The use according to any of claims 1 to 6, wherein the modulator is a CD100 or CD100 receptor nucleic acid.

13. The use according to any of claims 1 to 6, wherein the modulator is a CD100 or CD100 receptor protein.

14. The use according to any of claims 1 to 6, wherein the modulator is an agent that activates CD100 or CD100 receptor activity through direct interaction with the CD100 protein or CD100 receptor protein.

15. The use according to any of claims 1 to 6, wherein the medicament is in a form suitable for a direct injection into the brain.

16. An *in vitro* method for diagnosis or determination of the evolution of a condition involving a demyelination, which method comprises detecting in a biological sample of a test subject, the expression of a CD100 semaphorin or of a receptor thereof, compared with the expression of a CD100 semaphorin or of a receptor thereof in a biological sample of a control subject, wherein an increased expression of CD100 or of a receptor thereof is indicative of said condition or of a poor prognosis.

17. The method of claim 16, wherein the expression of the CD100 semaphorin or of a receptor thereof is determined by an immunoassay.

18. A pharmaceutical composition comprising CD100-/- oligodendrocytes or precursor cells thereof, wherein CD100 gene is inactived, in association with a pharmaceutically acceptable carrier.

19. Use of CD100-/- oligodendrocytes or precursor cells thereof, wherein CD100 gene is inactived, for the preparation of a medicament useful for axonal remyelination.

20. A method for inducing a demyelination in a non-human animal model, which method comprises administering to the animal model a CD100 semaphorin or a biologically active fragment thereof.
